# EUROPEAN PATENT SPECIFICATION

(11) **EP 2 986 120 B1**
(45) Date of publication and mention of the grant of the patent: **13.12.2017**
(21) Application number: 14784651.3
(22) Date of filing: 15.04.2014
(51) Int. Cl.: C07H 19/14, C07H 19/16, A61K 31/70, A61P 35/00, A01N 43/04

(54) **METHYLTRANSFERASE INHIBITORS FOR TREATING CANCER**
METHYLTRANSFERASEHEMMER ZUR BEHANDLUNG VON KREBS
INHIBITEURS DE MÉTHYLTRANSFÉRASE POUR LE TRAITEMENT DU CANCER

(30) Priority: 16.04.2013 US 201361812393 P
(43) Date of publication of application: 24.02.2016
(73) Proprietor: Sloan-kettering Institute For Cancer Research, New York, NY 10065 (US)
(72) Inventor: LUO, Minkui, New York, New York 10075 (US); ZHENG, Weihong, New York, New York 10075 (US)
(74) Representative: Cabinet Laurent & Charras
(86) International application number: PCT/US2014/034118
(87) International publication number: WO 2014/172330

(56) References cited:
- WO-A1-2012/050532
- WO-A1-2013/063417
- US-A1- 2008 132 525
- WEIHONG ZHENG ET AL: "Sinefungin Derivatives as Inhibitors and Structure Probes of Protein Lysine Methyltransferase SETD2", JOURNAL OF THE AMERICAN CHEMICAL SOCIETY, vol. 134, no. 43, 31 October 2012 (2012-10-31), pages 18004-18014, XP055049439, ISSN: 0002-7863, DOI: 10.1021/ja307060p
- LERNER C ET AL: "Bisubstrate inhibitors for the enzyme catechol-O-methyltransferase (COMT): influence of inhibitor preorganisation and linker length between the two substrate moieties on binding affinity", ORGANIC & BIOMOLECULAR CHEMISTRY, ROYAL SOCIETY OF CHEMISTRY, GB, vol. 1, no. 1, 1 January 2003 (2003-01-01) , pages 42-49, XP002343639, ISSN: 1477-0520, DOI: 10.1039/B208690P
- ZHENG, W ET AL.: 'Sinefungin Derivatives As Inhibitors And Structure Probes Of Protein Lysine Methyltransferase SETD2.' JACS. vol. 134, 08 October 2012, pages 18004 - 180014, XP055049439
- ANGLIN, J ET AL.: 'Synthesis And Structure-Activity Relationship Investigation Of Adenosine-Containing Inhibitors Of Histone Methyltransferase DOT1L.' JOURNAL OF MEDICINAL CHEMISTRY vol. 55, 27 August 2012, pages 8066 - 8074, XP055187162

### SEQUENCE LISTING

<110> Luo, Minkui
   Zheng, Weihong
<120> Methyltransferase Inhibitors for Treating Cancer
<130> 3314.032AWO
<150> US 61/812,393
   <151> 2013-04-16
<160> 7
<170> PatentIn version 3.5
<210> 1
   <211> 21
   <212> PRT
   <213> Artificial Sequence
<220>
   <223> synthetic peptide
<400> 1
   Ala Arg Thr Lys Gln Thr Ala Arg Lys Ser Thr Gly Gly Lys Ala Pro
   1               5                   10                  15
   Arg Lys Gln Leu Ala
               20
<210> 2
   <211> 19
   <212> PRT
   <213> Artificial Sequence
<220>
   <223> synthetic peptide
<400> 2
   Gly Gly Arg Gly Gly Phe Gly Gly Arg Gly Gly Phe Gly Gly Arg Gly
   1               5                   10                  15
   Gly Phe Gly
<210> 3
   <211> 40
   <212> PRT
   <213> Artificial Sequence
<220>
   <223> synthetic peptide
<400> 3
   Ala Arg Thr Lys Gln Thr Ala Arg Lys Ser Thr Gly Gly Lys Ala Pro
   1               5                   10                  15
   Arg Lys Gln Leu Ala Thr Lys Ala Ala Arg Lys Ser Ala Pro Ala Thr
               20                  25                  30
   Gly Gly Val Lys Lys Pro His Arg
           35                  40
<210> 4
   <211> 21
   <212> PRT
   <213> Artificial Sequence
<220>
   <223> synthetic peptide
<400> 4
   Leu Gly Lys Gly Gly Ala Lys Arg His Arg Lys Val Leu Arg Asp Asn
   1               5                   10                  15
   Ile Gln Gly Ile Thr
               20
<210> 5
   <211> 30
   <212> PRT
   <213> Artificial Sequence
<220>
   <223> synthetic peptide
<400> 5
   Ala Thr Lys Ala Ala Arg Lys Ser Ala Pro Ala Thr Gly Gly Val Lys
   1               5                   10                  15
   Lys Pro His Arg Tyr Arg Pro Gly Thr Val Ala Leu Arg Glu
               20                  25                  30
<210> 6
   <211> 34
   <212> PRT
   <213> Artificial Sequence
<220>
   <223> synthetic peptide
<400> 6
   Gly Gly Ser Arg Ala His Ser Ser His Leu Lys Ser Lys Lys Gly Gln
   1               5                   10                  15
   Ser Thr Ser Arg His Lys Lys Leu Met Phe Lys Thr Glu Gly Pro Asp
               20                  25                  30
   Ser Asp
<210> 7
   <211> 350
   <212> PRT
   <213> Artificial Sequence
<220>
   <223> synthetic peptide
<400> 7
   Met Asp Asp Gln Gln Ala Leu Asn Ser Ile Met Gln Asp Leu Ala Val
   1               5                   10                  15
   Leu His Lys Ala Ser Arg Pro Ala Leu Ser Leu Gln Glu Thr Arg Lys
               20                  25                  30
   Ala Lys Ser Ser Ser Pro Lys Lys Gln Asn Asp Val Arg Val Lys Phe
           35                  40                  45
   Glu His Arg Gly Glu Lys Arg Ile Leu Gln Phe Pro Arg Pro Val Lys
       50                  55                  60
   Leu Glu Asp Leu Arg Ser Lys Ala Lys Ile Ala Phe Gly Gln Ser Met
   65                  70                  75                  80
   Asp Leu His Tyr Thr Asn Asn Glu Leu Val Ile Pro Leu Thr Thr Gln
                   85                  90                  95
   Asp Asp Leu Asp Lys Ala Val Glu Leu Leu Asp Arg Ser Ile His Met
               100                 105                 110
   Lys Ser Leu Lys Ile Leu Leu Val Ile Asn Gly Ser Thr Gln Ala Thr
           115                 120                 125
   Asn Leu Glu Pro Leu Pro Ser Leu Glu Asp Leu Asp Asn Thr Val Phe
       130                 135                 140
   Gly Ala Glu Arg Lys Lys Arg Leu Ser Ile Ile Gly Pro Thr Ser Arg
   145                 150                 155                 160
   Asp Arg Ser Ser Pro Pro Pro Gly Tyr Ile Pro Asp Glu Leu His Gln
                   165                 170                 175
   Val Ala Arg Asn Gly Ser Phe Thr Ser Ile Asn Ser Glu Gly Glu Phe
               180                 185                 190
   Ile Pro Glu Ser Met Asp Gln Met Leu Asp Pro Leu Ser Leu Ser Ser
           195                 200                 205
   Pro Glu Asn Ser Gly Ser Gly Ser Cys Pro Ser Leu Asp Ser Pro Leu
       210                 215                 220
   Asp Gly Glu Ser Tyr Pro Lys Ser Arg Met Pro Arg Ala Gln Ser Tyr
   225                 230                 235                 240
   Pro Asp Asn His Gln Glu Phe Ser Asp Tyr Asp Asn Pro Ile Phe Glu
                   245                 250                 255
   Lys Phe Gly Lys Gly Gly Thr Tyr Pro Arg Arg Tyr His Val Ser Tyr
               260                 265                 270
   His His Gln Glu Tyr Asn Asp Gly Arg Lys Thr Phe Pro Arg Ala Arg
           275                 280                 285
   Arg Thr Gln Gly Thr Ser Leu Arg Ser Pro Val Ser Phe Ser Pro Thr
       290                 295                 300
   Asp His Ser Leu Ser Thr Ser Ser Gly Ser Ser Ile Phe Thr Pro Glu
   305                 310                 315                 320
   Tyr Asp Asp Ser Arg Ile Arg Arg Arg Gly Ser Asp Ile Asp Asn Pro
                   325                 330                 335
   Thr Leu Thr Val Met Asp Ile Ser Pro Pro Ser Arg Ser Pro
               340                 345                 350

## Description

### FIELD OF THE INVENTION

The invention relates to chemical compounds having methyltransferase inhibitory activity and their compositions for use in the treatment of diseases and conditions associated with inappropriate methyltransferase activity.

### BACKGROUND OF THE INVENTION

Epigenetics is inheritable information not encoded in DNA manifested through control of gene expression, thereby controlling a range of cellular activity, including determining cell fate, stem cell fate and regulating proliferation. Epigenetic control over gene expression is accomplished in at least four ways: (1) covalent histone modification, (2) covalent DNA modification, (3) histone variation, and (4) nucleosome structure and DNA/histone contact points. Epigenetic control through one mechanism can influence the other suggesting a combinatorial regulation, as evidenced by the methylation of histones being implicated in the modulation of DNA methylation.

Covalent histone modifications, a key mechanism involved in epigenetic control, include: (1) lysine acetylation, (2) lysine and arginine methylation, (3) serine and threonine phosphorylation, (4) ADP-ribosylation, (5) ubiquitination, and (6) SUMOylation. Specific enzymatic activities are associated with these modifications and in the case of histone methylation, methyltransferases catalyze the transfer of a methyl group from cofactor S-adenosylmethionine to a lysine or arginine, producing S-adenosylhomocysteine as a byproduct. Methyltransferases can also modify residues in other cellular proteins, e.g. the tumor suppressor p53.

Histone methyltransferases fall into subgroups that include arginine methyltransferases, SET-domain containing methyltransferases SU(VAR)3-9, E(Z) and TRX, and DOT-like methyltransferase hDOT1L. Families of SET-domain containing methyltransferases have been identified and include SUV39, SET1, SET2 and RIZ.

The disruption of the normal functions of methyltransferases has been implicated in human diseases. Members of different classes of methyltransferases are implicated in cancer and representative examples for the subgroups and subclasses are provided: (1) hDOT1L, a member of the DOT-like methyltransferases, is linked to leukemogenesis [Nature Cell Biology, 8:1017-1028 (2006); Cell, 121:167-178 (2005); Cell, 112:771-723 (2003)]. (2) EZH2, a SET1 methyltransferase, is up-regulated in tumor cell lines and has been linked to breast, gastric and prostate cancers [British Journal of Cancer, 90:761-769 (2004)]. (3) SUV39-1/2, SUV39 methyltransferases, have been linked to signaling pathways regulating cancer cell growth and differentiation [Genetica, 117(2-3):149-58 (2003)]. (4) NSD1, a SET2 subclass methyltransferase, has been linked to acute myeloid leukemia and Sotos syndrome, a predisposition to cancer [Molecular Cell Biology, 24(12):5184-96 (2004)]. (5) EVI1, a RIZ methyltransferase, is overexpressed in solid tumors and leukemia [Proceeding of the National Academy of Sciences, 93:1642-1647 (1996)]. (6) Related enzymes, namely SMYD2, are lysine methyltransferases that modify the tumor suppressor protein, p53 and through this activity, may function as an oncogene that interferes with p53's protective functions [Nature, 444(7119):629-632 (2006)]. (7) SMYD3, a SET-domain containing lysine methyltransferase, is involved in cancer cell proliferation [Nature Cell Biology, 6(8):731-740 (2004)]. (8) CARM1 (also known as PRMT4), an arginine methlytransferase, is linked to prostate cancer [Prostate, 66(12):1292-301 (2006)], breast cancer [Wang et al., Cancer Cell 25, 21-36, (2014)] and to myeloid leukemia [Vu et al., Cell Reports 5,1625-1638, (2013)].

Inappropriate methyltransferase activities thus represent attractive targets for therapeutic intervention by small molecule inhibitors. In fact, inhibitors of SUV(AR) histone methyltransferase [Nature Chemical Biology, 1:143-145 (2005)] and protein arginine methyltransferase [Journal of Biological Chemistry, 279:23892-23899 (2004)] have been described. The present invention relates to novel synthetic compounds effective as inhibitors of inappropriate histone methyltransferase activities. As a consequence of their inhibition of histone methyltransferase activity, these compounds would be useful in treating human diseases, such as cancer, particularly breast cancer, prostate cancer and hematological malignancies, such as leukemias and lymphomas, e.g. acute and chronic lymphoblastic and myelogenous leukemia, as well as Hodgkin's and non-Hodgkin's lymphomas.

Zheng et al. (Journal of The American Chemical Society, Vol. 134, no 34, 2012, pp 18004-18014) disclose adenosine derivatives as inhibitors of a methyltransferase enzyme, which are useful in the treatment of cancer.

### SUMMARY OF THE INVENTION

In one aspect, the invention relates to compounds of general formula I, which are potent and selective inhibitors of lysine and arginine methyltransferases: 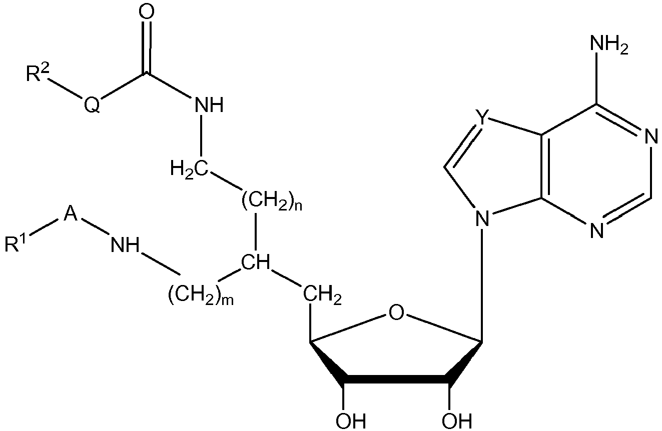 wherein:
Y is N or CH;
Q is NH or O;
A is chosen from direct bond, (C₁-C₂₀)hydrocarbon, (C₁-C₂₀)oxaalkyl and (C₁-C₂₀)azaalkyl;
R¹ is chosen from hydrogen, -C(=NH)NH₂, -C(=NH)NH(C₁-C₁₀)hydrocarbon, fluoro(C₁-C₆)hydrocarbon, and -CH(NH₂)COOH;
R² is R³, or when Q is NH, R² may additionally be -COR³ or -COOR³;
R³ is chosen from H, (C₁-C₂₀) hydrocarbon, substituted aryl, heteroaryl and substituted heteroaryl; m is 0, 1 or 2;
   and
n is 1, 2 or 3.

In these compounds, A is a bivalent moiety and R¹ is a substituent on A. The members of this genus are effective as inhibitors of methyltransferase activities and therefore, are useful for the inhibition, prevention and suppression of various pathologies associated with such activities, such as, for example, cancer cell and cancer stem cell fate differentiation, and cancer cell proliferation and cell cycle regulation. The compounds are also useful research tools for studying protein methyl transferase biology.

In another aspect, the invention relates to pharmaceutical compositions comprising a therapeutically effective amount of at least one compound of general formula I and a pharmaceutically acceptable carrier.

In another aspect, the invention relates to a therapeutically effective amount of a compound of formula I for use in the treatment of cancer.

### DETAILED DESCRIPTION OF THE INVENTION

Throughout this specification the substituents are defined when introduced and retain their definitions.

In one aspect, the invention relates to compounds having general formula I:

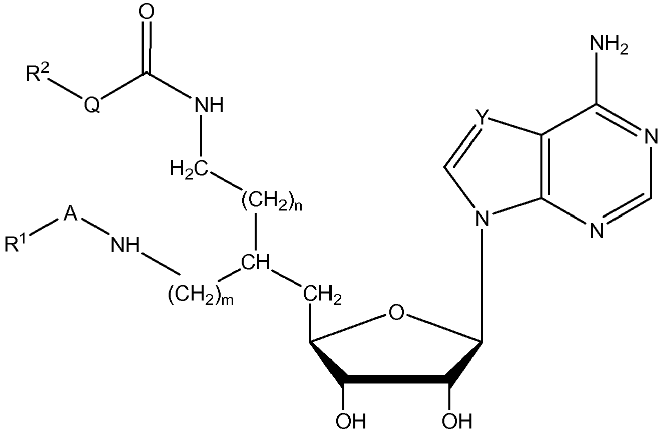

In some embodiments, R² is R³ and R³ is chosen from (C₁-C₆) alkyl and phenyl optionally substituted with one to three substituents chosen independently from halogen, haloalkyl, alkyl, acyl, hydroxyalkyl, hydroxy, alkoxy, haloalkoxy, oxaalkyl, carboxy, cyano, acetoxy, nitro, amino, alkylamino, dialkylamino, alkylthio, alkylsulfinyl, alkylsulfonyl, alkylsulfonylamino arylsulfonyl, arylsulfonylamino and benzyloxy. In some embodiments, R³ is chosen from (C₁-C₆) alkyl and *para*-monosubstituted phenyl.

In some embodiments n is 1. In some embodiments n is 3. In some embodiments n is 2 and m is 0 or 1.

In some embodiments, Q is NH; in others Q is O. Advantageously, Q is NH and R² is chosen from (C₁-C₆) alkyl, (C₃-C₇) cycloalkyl and phenyl substituted with one to three substituents chosen independently from halogen, haloalkyl, alkyl, acyl, hydroxyalkyl, hydroxy, alkoxy, and haloalkoxy. More advantageously, R² is phenyl substituted with halogen, halo(C₁-C₆)alkyl, (C₁-C₆) alkyl, acyl, hydroxymethyl, hydroxy, (C₁-C₆) alkoxy, and halo(C₁-C₆)alkoxy; n is 2; m is 0 or 1; and R¹-A- is hydrogen or (C₁-C₂₀) hydrocarbon.

In some embodiments, R¹-A is chosen from (C₁-C₆)alkyl, benzyl and (C₃-C₆)oxaalkyl. In these embodiments, R¹ is conceptually H and A is, for example, -(CH₂CH₂CH₂)-; or R¹ is conceptually H and A is 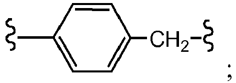 or R¹ is H and A is -(CH₂OCH₂CH₂CH₂)-. In other embodiments, R¹-A is chosen from hydrogen and -C(=NH)NH₂. In both these embodiments, A is a direct bond. Advantageously, R¹-A is chosen from hydrogen, benzyl and -C(=NH)NH₂.

In all of the foregoing embodiments, Y may be CH, i.e. the heterocycle is 7-deazapurine (also known as 7*H*-pyrrolo[2,3-*d*]pyrimidine) or Y may be N, i.e. the heterocycle is purine.

In another aspect, the invention relates to a pharmaceutical composition comprising a pharmaceutically acceptable carrier and the disclosed compound. In another aspect, the invention relates to the disclosed compound for use in the treatment of cancer, advantageously breast cancer or prostate cancer or a hematological malignancy such as leukemia. In another aspect, the invention relates to an *in vitro* or *ex vivo* method for inhibiting the activity of a methyltransferase enzyme comprising bringing said methyltransferase enzyme into contact with the disclosed compound. In a further aspect, the invention relates to an *in vitro* or *ex vivo* method for selectively inhibiting the activity of a first methyltransferase enzyme in the presence of a second methyltransferase enzyme comprising bringing both of said methyltransferase enzymes into contact with the disclosed compound.

For convenience and clarity certain terms employed in the specification, examples and claims are described herein.

Unless otherwise specified, alkyl (or alkylene) is intended to include linear or branched saturated hydrocarbon structures and combinations thereof. Alkyl refers to alkyl groups of from 1 to 20 carbon atoms, preferably 1 to 10 carbon atoms, more preferably 1 to 6 carbon atoms. Examples of alkyl groups include methyl, ethyl, propyl, isopropyl, n-butyl, s-butyl, t-butyl and the like. Lower alkyl refers to alkyl groups of from 1 to 4 carbon atoms.

Cycloalkyl is a subset of hydrocarbon and includes cyclic hydrocarbon groups of from 3 to 8 carbon atoms. Examples of cycloalkyl groups include c-propyl, c-butyl, c-pentyl, norbornyl and the like.

C₁ to C₂₀ hydrocarbon includes alkyl, cycloalkyl, polycycloalkyl, alkenyl, alkynyl, aryl and combinations thereof. Examples include benzyl, phenethyl, cyclohexylmethyl, adamantyl, camphoryl and naphthylethyl. Hydrocarbon refers to any substituent comprised of hydrogen and carbon as the only elemental constituents.

Unless otherwise specified, the term "carbocycle" is intended to include ring systems in which the ring atoms are all carbon but of any oxidation state. Thus (C₃-C₁₂) carbocycle refers to both non-aromatic and aromatic systems, including such systems as cyclopropane, benzene and cyclohexene. Carbocycle, if not otherwise limited, refers to monocycles, bicycles and polycycles. (C₈-C₁₂) Carbopolycycle refers to such systems as norbornane, decalin, indane and naphthalene

Alkoxy or alkoxyl refers to groups of from 1 to 20 carbon atoms, preferably 1 to 10 carbon atoms, more preferably 1 to 6 carbon atoms of a straight or branched configuration attached to the parent structure through an oxygen. Examples include methoxy, ethoxy, propoxy, isopropoxy and the like. Lower-alkoxy refers to groups containing one to four carbons. For the purpose of this application, alkoxy and lower alkoxy include methylenedioxy and ethylenedioxy.

Oxaalkyl refers to alkyl residues in which one or more carbons (and their associated hydrogens) have been replaced by oxygen. Examples include methoxypropoxy, 3,6,9-trioxadecyl and the like. The term oxaalkyl is intended as it is understood in the art [see Naming and Indexing of Chemical Substances for Chemical Abstracts, published by the American Chemical Society, 2002 edition, ¶196, but without the restriction of 127(a)], i.e. it refers to compounds in which the oxygen is bonded via a single bond to its adjacent atoms (forming ether bonds); it does not refer to doubly bonded oxygen, as would be found in carbonyl groups. Similarly, thiaalkyl and azaalkyl refer to alkyl residues in which one or more carbons has been replaced by sulfur or nitrogen, respectively. Examples of azaalkyl include ethylaminoethyl and aminohexyl.

As used herein, the term "optionally substituted" may be used interchangeably with "unsubstituted or substituted". The term "substituted" refers to the replacement of one or more hydrogen atoms in a specified group with a specified radical. For example, substituted alkyl, aryl, cycloalkyl, heterocyclyl etc. refer to alkyl, aryl, cycloalkyl, or heterocyclyl wherein one or more H atoms in each residue are replaced with halogen, haloalkyl, alkyl, acyl, alkoxyalkyl, hydroxyloweralkyl, carbonyl, phenyl, heteroaryl, benzenesulfonyl, hydroxy, loweralkoxy, haloalkoxy, oxaalkyl, carboxy, alkoxycarbonyl [-C(=O)O-alkyl], alkoxycarbonylamino [HNC(=O)O-alkyl], carboxamido [-C(=O)NH₂], alkylaminocarbonyl [-C(=O)NH-alkyl], cyano, acetoxy, nitro, amino, alkylamino, dialkylamino, (alkyl)(aryl)aminoalkyl, alkylaminoalkyl (including cycloalkylaminoalkyl), dialkylaminoalkyl, dialkylaminoalkoxy, heterocyclylalkoxy, mercapto, alkylthio, sulfoxide, sulfone, sulfonylamino, alkylsulfinyl, alkylsulfonyl, alkylsulfonylamino, arylsulfonyl, arylsulfonylamino, acylaminoalkyl, acylaminoalkoxy, acylamino, amidino, aryl, benzyl, heterocyclyl, heterocyclylalkyl, phenoxy, benzyloxy, heteroaryloxy, hydroxyimino, alkoxyimino, oxaalkyl, aminosulfonyl, trityl, amidino, guanidino, ureido, benzyloxyphenyl, and benzyloxy. "Oxo" is also included among the substituents referred to in "optionally substituted"; it will be appreciated by persons of skill in the art that, because oxo is a divalent radical, there are circumstances in which it will not be appropriate as a substituent (e.g. on phenyl). In one embodiment, 1, 2 or 3 hydrogen atoms are replaced with a specified radical. In the case of alkyl and cycloalkyl, more than three hydrogen atoms can be replaced by fluorine; indeed, all available hydrogen atoms could be replaced by fluorine. Such compounds (e.g.perfluoroalkyl) fall within the class of "fluorohydrocarbons". In preferred embodiments, substituents are halogen, haloalkyl, alkyl, acyl, hydroxyalkyl, hydroxy, alkoxy, haloalkoxy, oxaalkyl, carboxy, cyano, acetoxy, nitro, amino, alkylamino, dialkylamino, alkylthio, alkylsulfinyl, alkylsulfonyl, alkylsulfonylamino arylsulfonyl, arylsulfonylamino and benzyloxy.

Substituents Rⁿ are generally defined when introduced and retain that definition throughout the specification and in all independent claims.

As used herein, and as would be understood by the person of skill in the art, the recitation of "a compound" - unless expressly further limited - is intended to include salts of that compound. Thus, for example, the recitation "a compound of formula I" as depicted above, which incorporates a substituent COOH, would include salts in which the substituent is COO⁻ M⁺, wherein M is any counterion. Similarly, formula I as depicted above depicts a substituent NH₂, and therefore would also include salts in which the substituent is NH₃⁺ X⁻, wherein X is any counterion. Compounds containing a COOH substituent may commonly exist as zwitterions, which are effectively internal salts. In a particular embodiment, the term "compound of formula I" refers to the compound or a pharmaceutically acceptable salt thereof.

The term "pharmaceutically acceptable salt" refers to salts whose counter ion derives from pharmaceutically acceptable non-toxic acids and bases. Suitable pharmaceutically acceptable acids for salts of the compounds of the present invention include, for example, acetic, adipic, alginic, ascorbic, aspartic, benzenesulfonic (besylate), benzoic, boric, butyric, camphoric, camphorsulfonic, carbonic, citric, ethanedisulfonic, ethanesulfonic, ethylenediaminetetraacetic, formic, fumaric, glucoheptonic, gluconic, glutamic, hydrobromic, hydrochloric, hydroiodic, hydroxynaphthoic, isethionic, lactic, lactobionic, laurylsulfonic, maleic, malic, mandelic, methanesulfonic, mucic, naphthylenesulfonic, nitric, oleic, pamoic, pantothenic, phosphoric, pivalic, polygalacturonic, salicylic, stearic, succinic, sulfuric, tannic, tartaric acid, teoclatic, p-toluenesulfonic, and the like. Suitable pharmaceutically acceptable base addition salts for the compounds of the present invention include, but are not limited to, metallic salts made from aluminum, calcium, lithium, magnesium, potassium, sodium and zinc or organic salts made from lysine, arginine, N,N'-dibenzylethylenediamine, chloroprocaine, choline, diethanolamine, ethylenediamine, meglumine (N-methylglucamine) and procaine. Further pharmaceutically acceptable salts include, when appropriate, nontoxic ammonium cations and carboxylate, sulfonate and phosphonate anions attached to alkyl having from 1 to 20 carbon atoms.

It will be recognized that the compounds of this invention can exist in radio labeled form, i.e., the compounds may contain one or more atoms containing an atomic mass or mass number different from the atomic mass or mass number usually found in nature. Alternatively, a plurality of molecules of a single structure may include at least one atom that occurs in an isotopic ratio that is different from the isotopic ratio found in nature. Radioisotopes of hydrogen, carbon, phosphorous, fluorine, chlorine and iodine include ²H, ³H, ¹¹C, ¹³C, ¹⁴C, ¹⁵N, ³⁵S, ¹⁸F, ³⁶Cl, ¹²⁵I, ¹²⁴I and ¹³¹I respectively. Compounds that contain those radioisotopes and/or other radioisotopes of other atoms are within the scope of this invention. Tritiated, i.e. ³H, and carbon-14, i.e., ¹⁴C, radioisotopes are particularly preferred for their ease in preparation and detectability. Compounds that contain isotopes 11C, ¹³N, ¹⁵O, ¹²⁴I and ¹⁸F are well suited for positron emission tomography. Radiolabeled compounds of formula I of this invention and prodrugs thereof can generally be prepared by methods well known to those skilled in the art. Conveniently, such radiolabeled compounds can be prepared by carrying out the procedures disclosed in the Examples and Schemes by substituting a readily available radiolabeled reagent for a non-radio labeled reagent.

Persons of skill will readily appreciate that compounds described herein, when appropriately labeled as described above, can be employed in a method of identifying (i.e. labeling) specific methyltransferase enzymes in the presence of other enzymes, including other methyltransferase enzymes, for which their affinity is lower. Usually two orders of magnitude difference in affinity will be sufficient to distinguish between enzymes. Using methods well known to persons of skill in the art, specific methyltransferase enzymes can be localized in tissues, cells and organelles. A further aspect of the invention described herein is thus a method of identifying and/or localizing specific methyltransferase enzymes.

While it may be possible for the compounds of formula I to be administered as the raw chemical, it is preferable to present them as a pharmaceutical composition. According to a further aspect, the present invention provides a pharmaceutical composition comprising a compound of formula I or a pharmaceutically acceptable salt or solvate thereof, together with one or more pharmaceutically carriers thereof and optionally one or more other therapeutic ingredients. The carrier(s) must be "acceptable" in the sense of being compatible with the other ingredients of the formulation and not deleterious to the recipient thereof. The compositions may be formulated for oral, topical or parenteral administration. For example, they may be given intravenously, intraarterially, subcutaneously, and directly into the CNS - either intrathecally or intracerebroventricularly.

Formulations include those suitable for oral, parenteral (including subcutaneous, intradermal, intramuscular, intravenous and intraarticular), rectal and topical (including dermal, buccal, sublingual and intraocular) administration. The compounds are preferably administered orally or by injection (intravenous or subcutaneous). The precise amount of compound administered to a patient will be the responsibility of the attendant physician. However, the dose employed will depend on a number of factors, including the age and sex of the patient, the precise disorder being treated, and its severity. Also, the route of administration may vary depending on the condition and its severity. The formulations may conveniently be presented in unit dosage form and may be prepared by any of the methods well known in the art of pharmacy. In general, the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

It should be understood that in addition to the ingredients particularly mentioned above, the formulations of this invention may include other agents conventional in the art having regard to the type of formulation in question, for example those suitable for oral administration may include flavoring agents.

As used herein, "treatment" or "treating," or "palliating" or "ameliorating" are used interchangeably herein. These terms refers to an approach for obtaining a therapeutic benefit in the form of eradication or amelioration of the underlying disorder being treated. Also, a therapeutic benefit is achieved with the eradication or amelioration of one or more of the physiological systems associated with the underlying disorder such that an improvement is observed in the patient, notwithstanding that the patient may still be afflicted with the underlying disorder. The compositions may be administered to a patient at risk of developing a particular disease, or to a patient reporting one or more of the physiological symptoms of a disease, even though a diagnosis of this disease may not have been made.

Terminology related to "protecting", "deprotecting" and "protected" functionalities occurs throughout this application. Such terminology is well understood by persons of skill in the art and is used in the context of processes that involve sequential treatment with a series of reagents. In that context, a protecting group refers to a group which is used to mask a functionality during a process step in which it would otherwise react, but in which reaction is undesirable. The protecting group prevents reaction at that step, but may be subsequently removed to expose the original functionality. The removal or "deprotection" occurs after the completion of the reaction or reactions in which the functionality would interfere. Thus, when a sequence of reagents is specified, as it is in the processes described herein, the person of ordinary skill can readily envision those groups that would be suitable as "protecting groups". Suitable groups for that purpose are discussed in standard textbooks in the field of chemistry, such as Protective Groups in Organic Synthesis by T.W. Greene [John Wiley & Sons, New York, 1991].

A comprehensive list of abbreviations utilized by organic chemists appears in the first issue of each volume of the Journal of Organic Chemistry. The list is typically presented in a table entitled "Standard List of Abbreviations".

In general, the compounds of the present invention may be prepared by the methods illustrated in the general reaction schemes as, for example, described below, or by modifications thereof, using readily available starting materials, reagents and conventional synthesis procedures. In these reactions, it is also possible to make use of variants that are in themselves known, but are not mentioned here. The starting materials are either commercially available, synthesized as described in the examples or may be obtained by the methods well known to persons of skill in the art. The synthetic methods parallel those described in PCT application WO2013/063417.

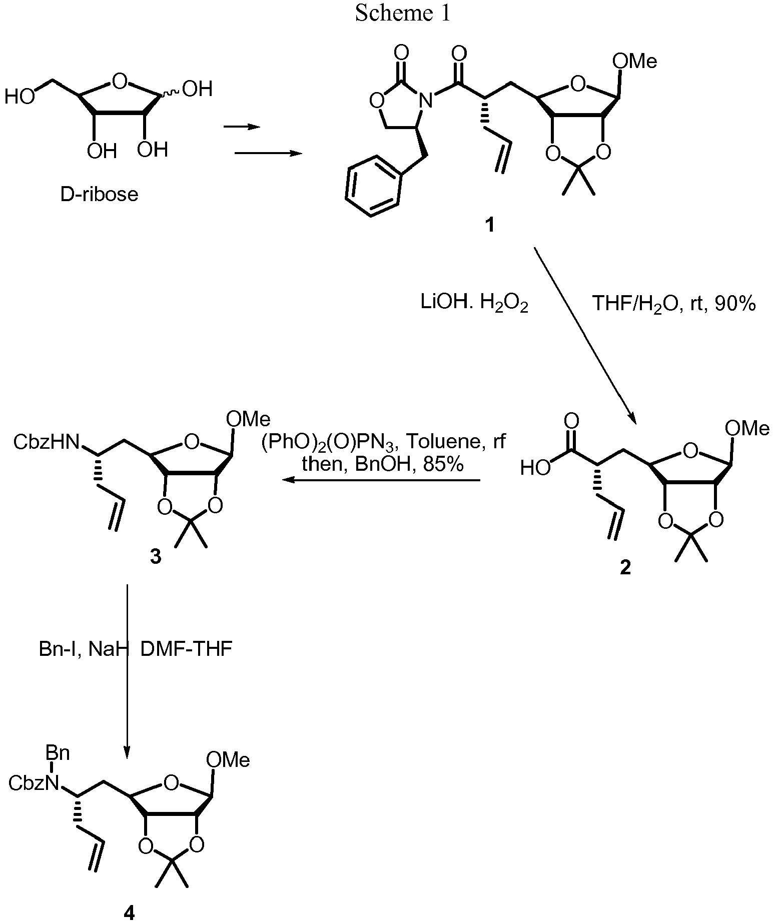

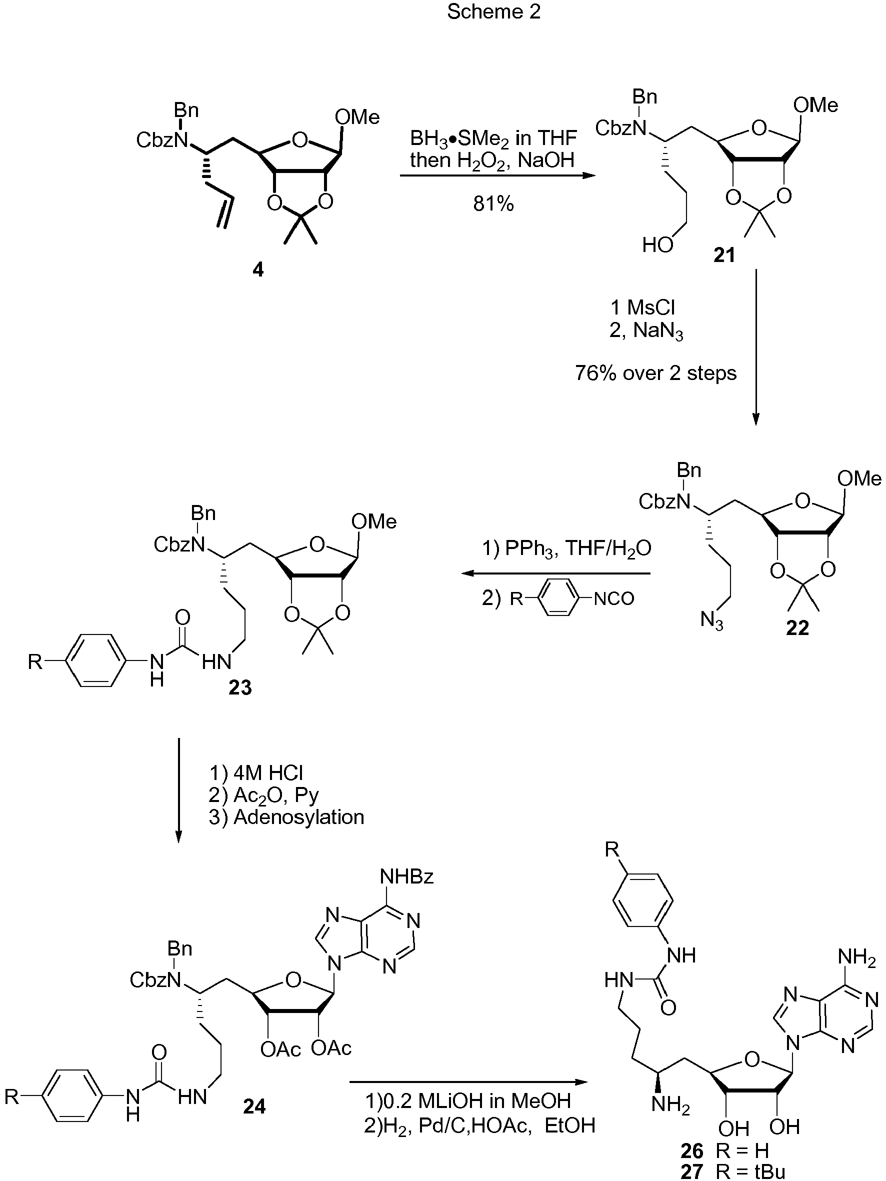

The synthesis of compounds **26** and **27** was started with intermediate **4.** The hydroboration of compound **4,** after oxidative work-up, gave primary alcohol. The conversion of alcohol into corresponding amino derivate was accomplished via azide substitution and Staudinger reduction. It was readily reacted with isocyanate to construct the urea. From this precursor, routine adenosylation and deprotection, which have been described in PCT application US12/62157, delivered target compounds.

Azide **22** ¹H NMR (CDCl3, 600MHz): δ1.11-1.22(m, 5H), 1.33-1.34(m, 0.5H), 1.39-1.42(m, 3.5H), 1.50-1.59(m, 3H), 1.74-1.78(m, 0.5H), 1.95-1.98(m, 1.5H), 2.80(s, 1H), 2.93(t, 1H, *J* = 6.6Hz), 3.17(s, 1.3H), 3.27(s, 1.7H), 4.11(dd, 0.6H, *J* = 10.8Hz, 3.3Hz), 4.15(d, 0.6H, *J* =15.4Hz), 4.23(d, 0.4H, *J* = 5.6Hz), 4.39(d, 0.6H, *J* = 5.8Hz), 4.44(d, 0.4H, *J* = 5.8Hz), 4.51(d, 0.4H, *J* = 5.8Hz), 4.60-4.62(m, 0.6H), 4.83(s, 0.4H), 4.89(s, 0.6H), 5.11-5.18(m, 2H), 7.16-7.32(m, 10H); ¹³C NMR (CDCl3, 600MHz *rotamers*): δ 24.87, 24.93, 25.29, 25.82, 25.96, 26.45, 29.73, 30.26, 31.07, 31.61, 34.68, 37.61, 38.69, 50.03, 50.59, 50.84, 50.91, 54.59, 54.93, 55.24, 55.41, 67.18, 67.64, 83.82, 83.92, 84.21, 84.33, 85.47, 85.53, 109.99, 110.18, 112.24, 112.31, 127.49, 127.97, 127.02, 128.14, 128.26, 128.38, 128.51, 128.54, 136.52, 138.52, 138.73, 155.70, 157.30. MS(ESI)m/z: 547 ([M+Na]⁺ ; HRMS: calculated for C₂₈H₃₆N₄O₆Na ([M+Na] ⁺) 547.2533, found 547.2518.

Compound **26**: MS(ESI)m/z: 457([M+H]⁺ ; HRMS: calculated for C₂₁H₂₉N₈O₄ ([M+H] ⁺) 457.2312, found 457.2316.

Compound **27**:¹H NMR (MeOD, 500MHz): δ 1.21(s, 9H), 1.54-1.57(m, 2H), 1.64-1.69(m, 2H), 2.01-2.06(m, 1H), 2.11-2.16(m, 1H), 3.12-.315(m, 2H), 3.37-3.41(m, 1H), 4.11-4.16(m, 1H), 4.20(t, 1H, *J* = 5.8Hz), 5.58(dd, 1H, *J* = 5.4HZ, 4Hz), 5.97(d, 1H, *J* = 3.8Hz), 7.16(d, 2H, *J* = 6.8Hz), 7.21(d, 2H, *J* = 5.8Hz), 8.34(s, 1H), 8.35(s, 1H); MS(ESI)m/z: 513([M+H]⁺ ; HRMS: calculated for C₂₅H₃₇N₈O₄ ([M+H] ⁺) 513.2938, found 513.2925.

Other compounds in which m is zero and n is two can be made in analogous fashion by substituting the appropriate isocyanate in the conversion of **22** to **23** in Scheme 2.

Compounds in which m is one and n is 1 can be made as shown in Scheme 3 below:

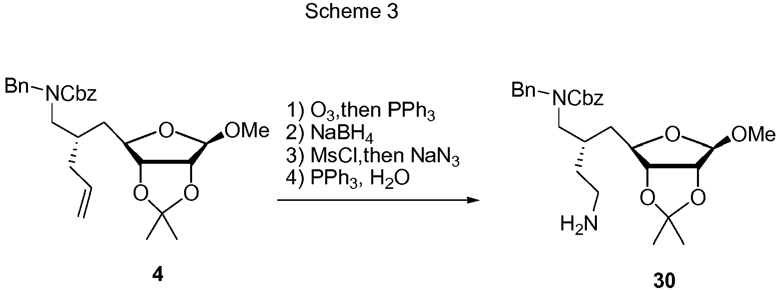

Compounds in which m is one and n is 3 can be made as shown in Scheme 4 below:

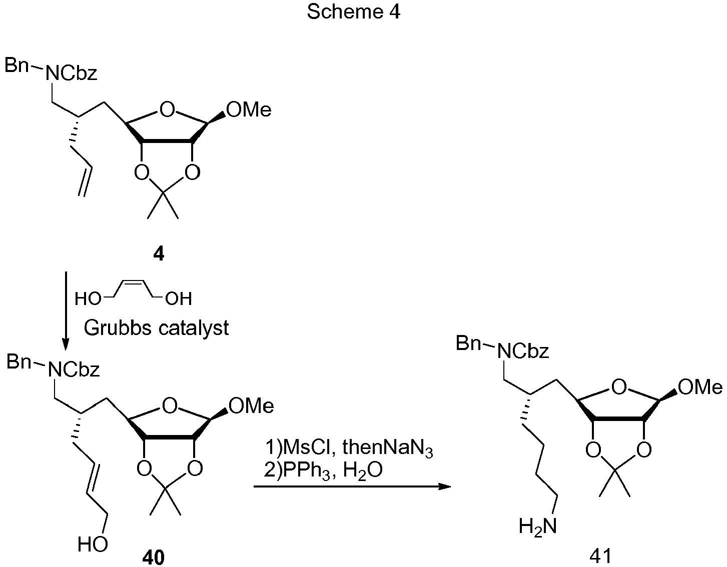

Compounds in which m is two can be made as shown in Scheme 5 below:

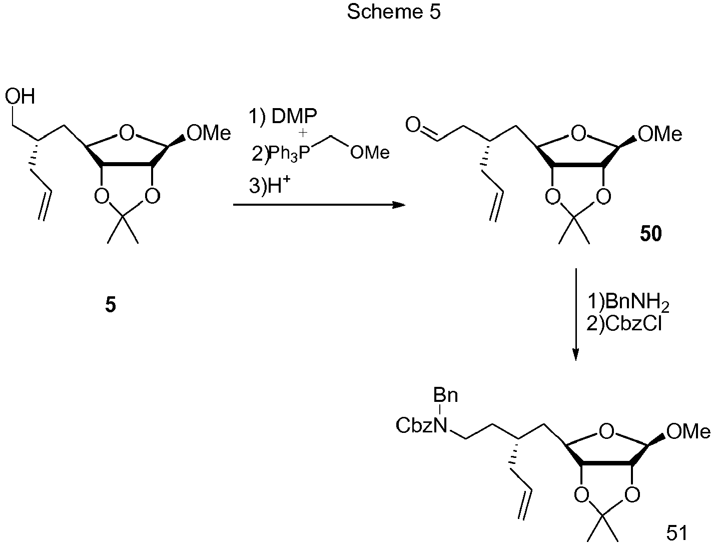

Compounds in which Y is CH may be synthesized by using the appropriate deazapurine as shown in Scheme 6:

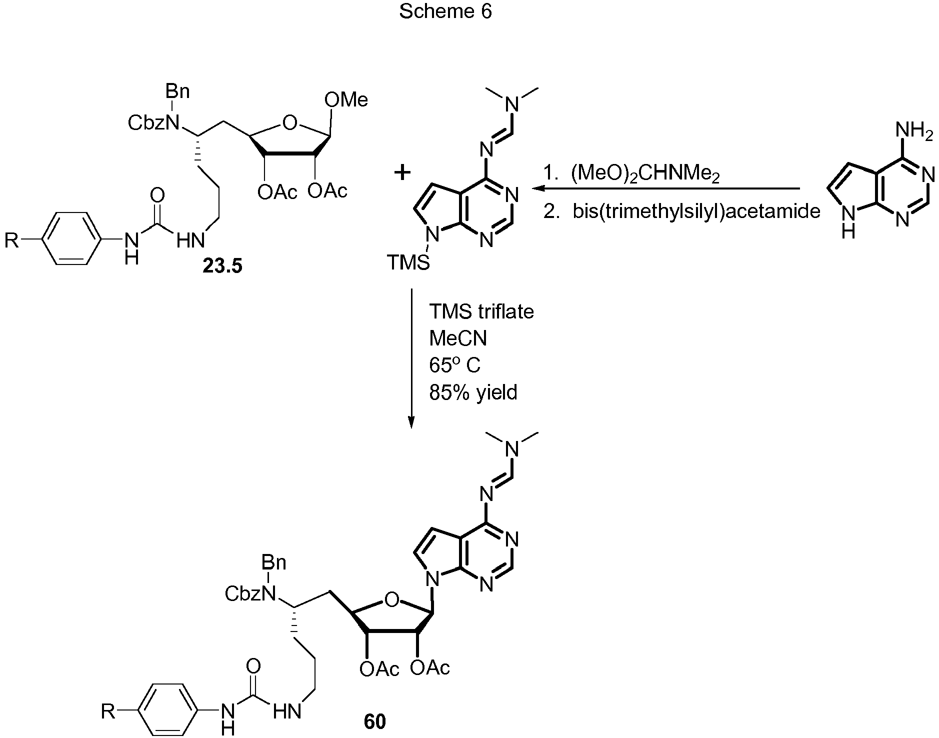

Compounds in which m is one and n is 2 can be made as shown in Scheme 7 below:

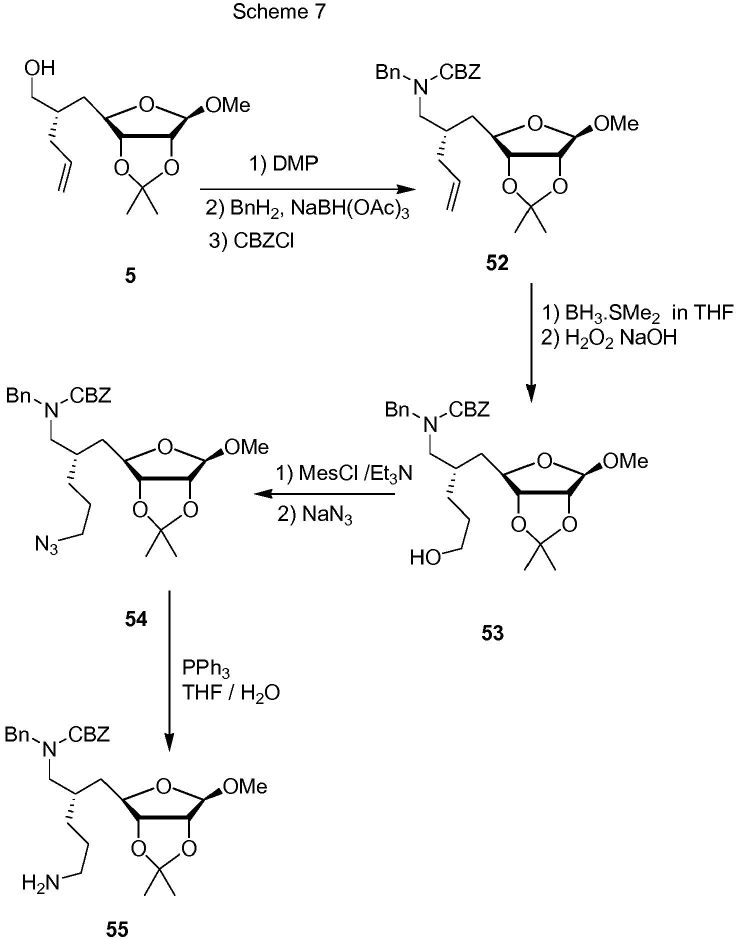

Two particular examples, 28 and 29, were made from intermediate 55 as shown:

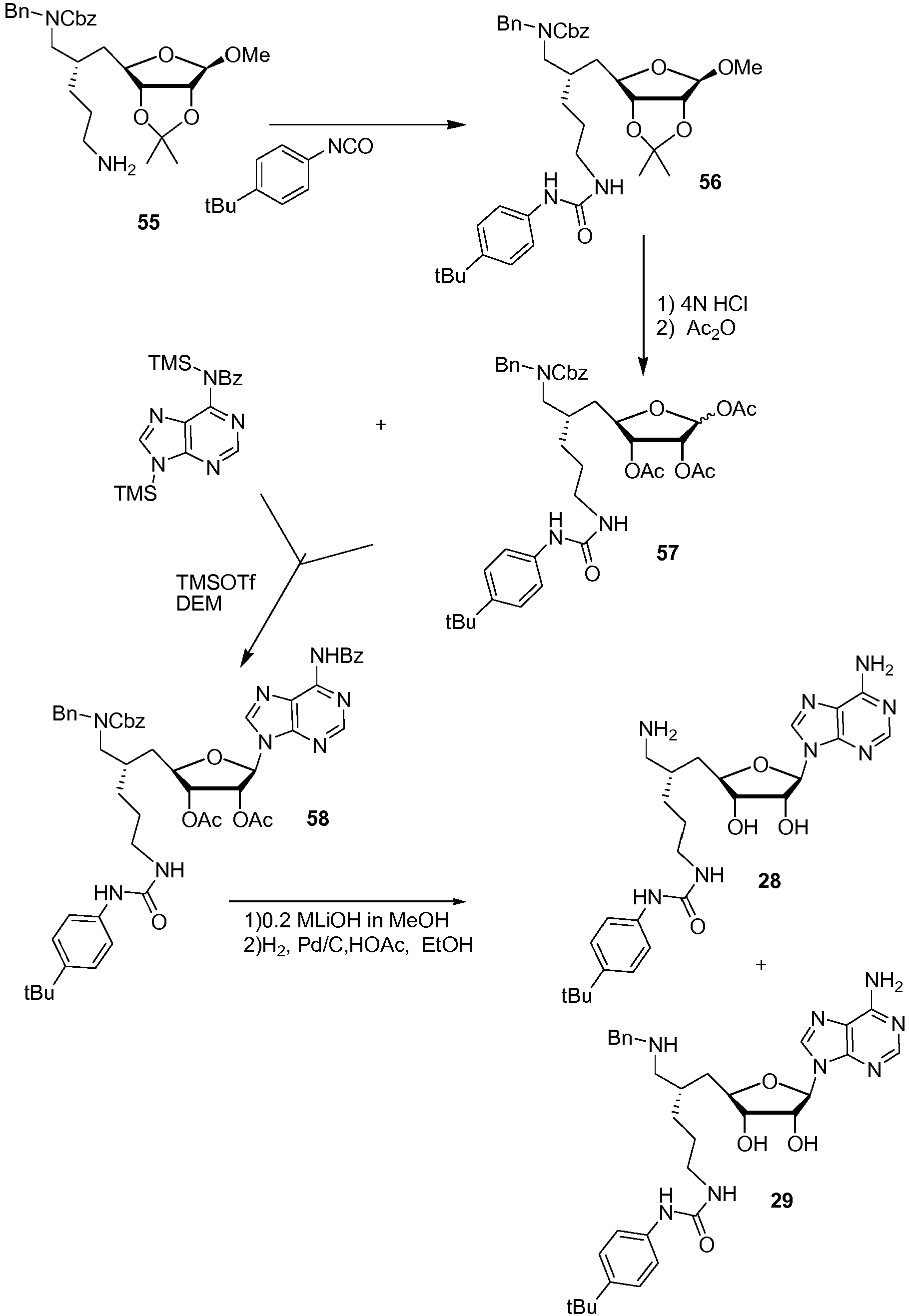

Example **28**: ¹H NMR (MeOD, 500MHz): δ 1.29(s, 9H), 1.40-1.65(m, 4H), 1.82-1.90(m, 1H), 1.95-2.05(m, 1H), 2.87 (dd, 1H, *J* = 12.5, 7.5), 3.01 (dd, 1H, *J* = 12.5, 7.5Hz), 3.12-.325(m, 3H), 4.11-4.16(m, 1H), 4.18(t, 1H, *J* = 5.5Hz), 4.72 (t, 1H, *J* = 5.5Hz), 5.97(d, 1H, *J* = 3.8Hz), 7.16(d, 2H, *J* = 8.7 Hz), 7.21(d, 2H, *J* = 8.7 Hz), 8.30(s, 1H), 8.32(s, 1H).

Example **29**: ¹H NMR (MeOD, 500MHz): δ 1.29(s, 9H), 1.42-1.63(m, 4H), 1.85-1.93(m, 1H), 2.02-2.15(m, 2H), 3.02 (dd, 1H, *J* = 12.5, 7.5), 3.05-3.18 (m, 3H), 3.19-3.25(m, 1H), 4.08-4.12(m, 3H), 4.19(t, 1H, *J* = 5.8Hz), 4.65 (t, 1H, *J* = 5.8Hz), 5.96(d, 1H, *J* = 3.8Hz), 7.20-7.40(m, 10H), 8.26(s, 1H), 8.31(s, 1H); HRMS: calculated for C₃₃H₄₅N₈O₄ ([M+H] ⁺) 617.3564, found 617.3549.

The compounds described above were tested as described below:

Methylation Reaction. The 20 µL methylation reaction was carried out at ambient temperature using two mixtures: A. 10 µl of enzyme mixture in the assay buffer containing 50 mM Hepes (pH=8.0), 0.005% Tween-20, 5µg/ml BSA and 1mM TCEP; B. 10 µl of a mixture of 1.5 µM, 0.15 µCi [³H-Me]-SAM cofactor and 3 µM of the corresponding peptide substrate in the same assay buffer. After A and B were mixed for a designated time period, the reaction mixture was examined with our filter-paper assay.

Conditions for the enzymes:

| Enzyme | [Enzyme mixture] (nM) | [Enzyme] _{final} (nM) | Substrate | Reaction Time (h) |
|---|---|---|---|---|
| G9a (913-1913) | 40 | 20 | H3 (1-21 aa) | 1 |
| GLP1 (951-1235) | 20 | 10 | H3 (1-21 aa) | 1 |
| SUV39H2 (112-410) | 10 | 5 | H3 (1-21 aa) | 4 |
| SET7/9 Full-length | 300 | 150 | H3 (1-21 aa) | 3 |
| PRMT1 (10-352) | 200 | 100 | RGG | 1.5 |
| PRMT3 (211-531) | 200 | 100 | RGG | 3 |
| CARM1 (19-608) | 600 | 300 | H3 (1-40 aa) | 7 |
| SET8 (191-352) | 2000 | 1000 | H4 (10-30 aa) | 8 |
| SETD2 (1347-1711) | 500 | 250 | H3 (20-50 aa) | 4 |
| SMYD2 Full-length | 100 | 50 | p53 (360-393 aa) | 10 |
| SMYD3 | 250 | 125 | MAP3K2 (1-350 aa) | 2 |
| SETDB1Full-length | 15 | 7.5 | H3 (1-21 aa) | 15 |
| DOT1L | 100 | 50 | Nucleosomes | 6 |

**H3 (1-21-aa):** ARTKQTARKSTGGKAPRKQLA (SEQ ID NO: 1)
**RGG:** GGRGGFGGRGGFGGRGGFG (SEQ ID NO: 2)
**H3 (1-40 aa):** ARTKQTARKSTGGKAPRKQLATKAARKSAPATGGVKKPHR (SEQ ID NO: 3)
**H4 (10-30 aa):** LGKGGAKRHRKVLRDNIQGIT (SEQ ID NO: 4)
**H3 (20-50 aa):** ATKAARKSAPATGGVKKPHRYRPGTVALRE (SEQ ID NO: 5)
**p53 (360-393 aa):** GGSRAHSSHLKSKKGQSTSRHKKLMFKTEGPDSD (SEQ ID NO: 6) **MAP3K2 (1-350 aa):**

Filter-paper Assay. This assay relies on Whatman P-81 filter paper, which binds peptides but not SAM. Protein Methyl Transferases (PMTs) transfer ³H-Me of [³H-Me]-SAM to peptide substrates and the resultant ³H-methylated, filter-paper-bound peptide is quantified with a scintillation counter. Briefly, 6 µl of the methylation reaction was spotted onto Whatman P-81 phosphocellulose filter paper (1.2 x 1.2 cm²) to immobilize the ³H-labeled peptide. After drying in air for 20 min, the filter paper was immersed into 20 mL of 50 mM Na₂CO₃/NaHCO₃ buffer (pH=9.2), and washed 5 times for 10 min each time. The washed filter paper was then transferred to a 20 ml scintillation vial containing 1 mL of distilled water and 10 mL of Ultima Gold scintillation cocktail or 7 mL scintillation vial containing 0.5 mL od distilled water and 5 mL of scintillation cocktail (PerkinElmer). The radioactivity was quantified by a Beckman LS6000IC liquid scintillation counter.

Dose-response Curves and IC₅₀. Twice the PMT concentration was incubated for 10 min with varied concentration of inhibitors (0.1 - 400 µM stocks), into which 10 µl of the PMT peptide substrate and radioactive cofactor (3 µM of the corresponding peptide and 1.5 µM, 0.15 µCi [³H-Me]-SAM) were added. After incubating the reaction mixture for the respective reaction time, the conversion was quantified with the filter paper assay as described above. The inhibition was expressed as the percentage between the high control (no inhibition) and the low control (no enzyme) as follows: Percentage Inhibition = [(high control - reading)/(high control - low control)] ×100%. Each experiment was performed in triplicate. The IC₅₀ values were obtained by fitting inhibition percentage versus inhibitor concentration using GraphPad Prism5 software.

The results are shown in the following table, in which S-adenosyl homocysteine (SAH) and sinefungin (SIN) are controls:

| | SAH | Sinefungin | Example 26 | Example 27 | Example 28 | Example 29 |
|---|---|---|---|---|---|---|
| **G9a** | 6.66 | 18.86 | >100 | >100 | >100 | >100 |
| **GLP1** | 5.03 | 32.02 | >100 | >100 | >100 | >100 |
| **SET7/9** | >100 | 1.14 | >100 | >100 | >100 | >100 |
| **SET8** | >100 | >100 | >100 | >100 | >100 | >100 |
| **SETD2** | 2.94 | 28.44 | 42.7 | 23.4 | >100 | >100 |
| **PRMT1(100nm, RGG)** | 8.59 | 1.034 | ∼100 | >100 | >100 | >100 |
| **PRMT3** | 39.5 | 28.17 | 5.9 | 35.4 | >50 | >50 |
| **SUV39H2** | 0.63 | 4.58 | >100 | >100 | >100 | ∼10 |
| **CARM1** | 1.90 | 0.44 | 1.9 | 0.1 | 0.73 | <0.2 |
| **SMYD2-FL** | ∼50 | 0.22 | 61.6 | ∼100 | >100 | >100 |
| **SMYD3** | | | | | >100 | >100 |
| **SETDB1-FL** | 0.95 | 8 | 55 | ∼100 | | |
| **DOT1L** | 2.2 | 53.4 | 0.84 | 0.14 | 0.07 | 0.17 |

Compounds 27, 28 and 29 showed remarkable inhibitory activity to CARM1 and to DOT1L. Compared to the positive control, the replacement of aminoacid with phenyl urea surprisingly did not have a deleterious effect on the affinity to some enzymes. Given that this replacement reduced the overall polarity of sinefungin, it is predicted that compounds of the invention will exhibit increased membrane permeability.

Compounds that show selective inhibition of one or a few families of PMTs are of greater interest as candidates for use in therapy, since it is believed that broad spectrum inhibition is likely to be associated with a higher probability of side effects. In this regard, compounds **27, 28** and **29** are of interest.

## Claims

1. A compound of formula I 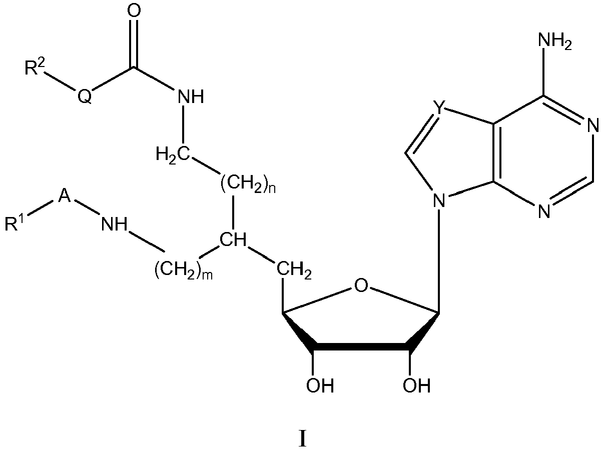 wherein:
Y is N or CH;
Q is NH or O;
A is chosen from direct bond, (C₁-C₂₀)hydrocarbon, (C₁-C₂₀)oxaalkyl and (C₁-C₂₀)azaalkyl;
R¹ is chosen from hydrogen, -C(=NH)NH₂, -C(=NH)NH(C₁-C₁₀)hydrocarbon, fluoro(C₁-C₆)hydrocarbon, and -CH(NH₂)COOH;
R² is R³, or when Q is NH, R² may additionally be -COR³ or -COOR³;
R³ is chosen from H, (C₁-C₂₀) hydrocarbon, substituted aryl, heteroaryl and substituted heteroaryl;
m is 0, 1 or 2;
and
n is 1, 2 or 3.

2. A compound according to claim 1 wherein R³ is chosen from (C₁-C₆) alkyl and phenyl optionally substituted with one to three substituents chosen independently from halogen, haloalkyl, alkyl, acyl, hydroxyalkyl, hydroxy, alkoxy, haloalkoxy, oxaalkyl, carboxy, cyano, acetoxy, nitro, amino, alkylamino, dialkylamino, alkylthio, alkylsulfinyl, alkylsulfonyl, alkylsulfonylamino, arylsulfonyl, arylsulfonylamino and benzyloxy.

3. A compound according to claim 2 wherein R³ is chosen from (C₁-C₆) alkyl and *para-*monosubstituted phenyl.

4. A compound according to claim 1 wherein n is 1 or 3.

5. A compound according to claim 1 wherein n is 2 and m is 0 or 1.

6. A compound according to claim 1 wherein Q is NH and R² is chosen from (C₁-C₆) alkyl, (C₃-C₇) cycloalkyl and phenyl substituted with one to three substituents chosen independently from halogen, haloalkyl, alkyl, acyl, hydroxyalkyl, hydroxy, alkoxy, and haloalkoxy.

7. A compound according to claim 6 wherein R² is phenyl substituted with halogen, halo(C₁-C₆)alkyl, (C₁-C₆) alkyl, acyl, hydroxymethyl, hydroxy, (C₁-C₆) alkoxy, and halo(C₁-C₆)alkoxy; n is 2; m is 0 or 1; and R¹-A- is hydrogen or (C₁-C₂₀) hydrocarbon.

8. A compound according to claim 1 wherein R¹-A is chosen from hydrogen, benzyl and -C(=NH)NH₂.

9. A compound according to any of claims 1 to 8 wherein Y is CH or N.

10. A pharmaceutical composition comprising a pharmaceutically acceptable carrier and a compound according to any of claims 1 to 9.

11. A method for inhibiting the activity of a methyltransferase enzyme comprising bringing said methyltransferase enzyme into contact with a compound according to any of claims 1 to 9, wherein the method is not practised on the human or animal body.

12. A method for selectively inhibiting the activity of a first methyltransferase enzyme in the presence of a second methyltransferase enzyme comprising bringing both of said methyltransferase enzymes into contact with a compound according to any of claims 1 to 9, wherein the method is not practised on the human or animal body.

13. A compound according to any of claims 1 to 9 for use in the treatment of cancer.

14. A compound for its use according to claim 13 wherein said cancer is breast cancer or prostate cancer.

15. A compound for its use according to claim 13 wherein said cancer is a hematological malignancy, advantageously leukemia

## Patentansprüche

1. Eine Verbindung mit der Formel I 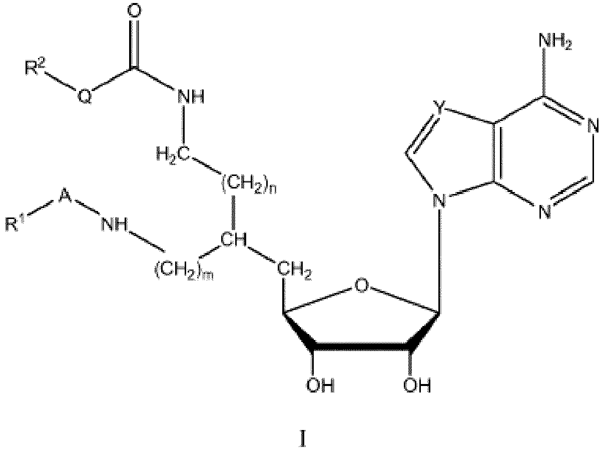 wobei
Y N oder CH ist;
Q NH oder O ist;
A aus der Direktbindung, (C₁-C₂₀) Kohlenwasserstoff, (C₁-C₂₀)Oxaalkyl und (C₁-C₂₀)Azaalkyl ausgewählt wird;
R¹ aus Wasserstoff, -C(=NH)NH₂, -C(=NH)NH(C₁-C₁₀) Kohlenwasserstoff, Fluoro(C₁-C₆) Kohlenwasserstoff, und -CH(NH₂)COOH ausgewählt wird;
R² R³ ist, oder wenn Q NH ist, kann R² zusätzlich -COR³ oder -COOR³ sein;
R³ aus H, (C₁-C₂₀) Kohlenwasserstoff, substituiertem Aryl, Heteroaryl und substituiertem Heteroaryl ausgewählt wird;
m 0, 1 oder 2 ist;
und
n 1, 2 oder 3 ist.

2. Eine Verbindung gemäß Anspruch 1 wobei R³ aus (C₁-C₆) Alkyl und Phenyl ausgewählt wird, optional ersetzt durch einen bis drei Substituenten, ausgewählt unabhängig aus Halogen, Haloalkyl, Alkyl, Acyl, Hydroxyalkyl, Hydroxy, Alkoxy, Haloalkoxy, Oxaalkyl, Carboxy, Cyano, Acetoxy, Nitro, Amino, Alkylamino, Dialkylamino, Alkylthio, Alkylsulfinyl, Alkylsulfonyl, Alkylsulfonylamino, Arylsulfonyl, Arylsulfonylamino und Benzyloxy.

3. Eine Verbindung gemäß Anspruch 2 wobei R³ aus (C₁-C₆) Alkyl und *para*monosubstitutiertem Phenyl ausgewählt wird.

4. Eine Verbindung gemäß Anspruch 1 wobei n 1 oder 3 ist.

5. Eine Verbindung gemäß Anspruch 1 wobei n 2 und m 0 oder 1 ist.

6. Eine Verbindung gemäß Anspruch 1 wobei Q NH ist und R² aus (C₁-C₆) Alkyl, (C₃-C₇)Cycloalkyl und Phenyl ausgewählt wird, substitutiert mit eins bis drei Substituenten, ausgewählt unabhängig aus Halogen, Haloalkyl, Alkyl, Acyl, Hydroxyalkyl, Hydroxy, Alkoxy und Haloalkoxy.

7. Eine Verbindung gemäß Anspruch 6 wobei R² mit Halogen, Halo(C₁-C₆)alkyl, (C₁-C₆) Alkyl, Acyl, Hydroxymethyl, Hydroxy, (C₁-C₆) Alkoxy, und Halo(C₁-C₆)alkoxy phenylsubstitutiert ist; n ist 2; m ist 0 oder 1; und R¹-A- ist Wasserstoff oder (C₁-C₂₀) Kohlenwasserstoff.

8. Eine Verbindung gemäß Anspruch 1 wobei R¹-A aus Wasserstoff, Benzyl und - C(=NH)NH₂ ausgewählt wird.

9. Eine Verbindung gemäß irgendeinem der Ansprüche 1 bis 8 wobei Y CH oder N ist.

10. Eine pharmazeutische Zusammensetzung, umfassend eine pharmazeutisch unbedenkliche Trägersubstanz und eine Verbindung gemäß irgendeinem der Ansprüche 1 bis 9.

11. Verfahren zur Hemmung der Aktivität eines Methyltransferase - Enzyms, das umfasst, dieses Methyltransferase - Enzym in Kontakt mit einer Verbindung nach irgendeinem der Ansprüche 1 bis 9 zu bringen, wobei dieses Verfahren nicht am menschlichen oder tierischen Körper vorgenommen wird.

12. Verfahren zur selektiven Hemmung der Aktivität eines ersten Methyltransferase - Enzyms, in Anwesenheit eines zweiten Methyltransferase - Enzyms, das umfasst, diese beiden Methyltransferase - Enzyme in Kontakt mit einer Verbindung nach irgendeinem der Ansprüche 1 bis 9 zu bringen, wobei dieses Verfahren nicht am menschlichen oder tierischen Körper vorgenommen wird.

13. Eine Verbindung gemäß irgendeinem der Ansprüche 1 bis 9 zur Verwendung bei der Krebsbehandlung.

14. Eine Verbindung zur Verwendung gemäß Anspruch 13 wobei es sich bei dieser Krebserkrankung um Brustkrebs oder Prostata-Krebs handelt.

15. Eine Verbindung zur Verwendung gemäß Anspruch 13 wobei es sich bei dieser Krebserkrankung um bösartige Bluterkrankungen, vorzugsweise Leukämie handelt.

## Revendications

1. Composé de formule I 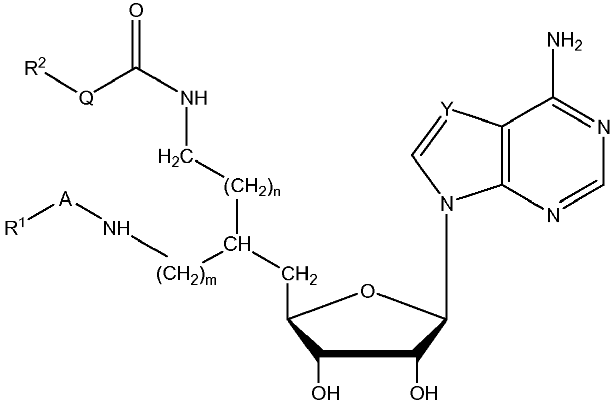 dans laquelle :
Y est N ou CH ;
Q est NH ou O ;
A est choisi parmi une liaison directe, un (C₁-C₂₀)hydrocarbone, un (C₁-C₂₀)oxaalkyl et un (C₁-C₂₀)azaalkyl ;
R¹ est choisi parmi l'hydrogène, -C(=NH)NH₂, un -C(=NH)NH(C₁-C₁₀)hydrocarbone, un fluoro(C₁-C₆)hydrocarbone, et -CH(NH₂)COOH ;
R² est R³, ou quand Q est NH, R² peut également être -COR³ ou -COOR³ ;
R³ est choisi parmi H, un (C₁-C₂₀)hydrocarbone, un aryl substitué, un hétéroaryl et un hétéroaryl substitué ;
m est 0, 1 ou 2 ;
et
n est 1, 2 ou 3.

2. Composé selon la revendication 1 dans lequel R³ est choisi parmi un (C₁-C₆)alkyl et un phényl optionnellement substitué avec un à trois substituants choisis indépendamment parmi un halogène, un haloalkyl, alkyl, un acyl, un hydroxyalkyl, un hydroxy, un alkoxy, un haloalkoxy, un oxaalkyl, un carboxy, un cyano, un acetoxy, un nitro, un amino, un alkylamino, un dialkylamino, un alkylthio, un alkylsulfinyl, un alkylsulfonyl, un alkylsulfonylamino, un arylsulfonyl, un arylsulfonylamino et un benzyloxy.

3. Composé selon la revendication 2 dans lequel R³ est choisi parmi un (C₁-C₆)alkyl et un phényl *para*-monosubstitué.

4. Composé selon la revendication 1 dans lequel n est 1 ou 3.

5. Composé selon la revendication 1 dans lequel n est 2 et m est 0 ou 1.

6. Composé selon la revendication 1 dans lequel Q est NH et R² est choisi parmi un (C₁-C₆)alkyl, un (C₃-C₇)cycloalkyl et un phényl substitué avec un à trois substituants choisis indépendamment parmi un halogène, un haloalkyl, un alkyl, un acyl, un hydroxyalkyl, un hydroxy, un alkoxy et un haloalkoxy.

7. Composé selon la revendication 6 dans lequel R² est un phényl substitué avec un halogène, un halo(C₁-C₆)alkyl, un (C₁-C₆)alkyl, un acyl, un hydroxyméthyl, un hydroxy, un (C₁-C₆)alkoxy et un halo(C₁-C₆)alkoxy ; n est 2 ; m est 0 ou 1 ; et R¹ -A- est un hydrogène ou un (C₁-C₂₀)hydrocarbone.

8. Composé selon la revendication 1 dans lequel R¹-A- est choisi parmi un hydrogène, un benzyl et -C(=NH)NH₂.

9. Composé selon l'une quelconque des revendications 1 à 8 dans lequel Y est CH ou N.

10. Composition pharmaceutique comprenant un vecteur pharmaceutiquement acceptable et un composé selon l'une quelconque des revendications 1 à 9.

11. Méthode d'inhibition de l'activité de l'enzyme méthyltransférase comprenant la mise en contact de ladite enzyme méthyltransférase avec un composé selon l'une quelconque des revendications 1 à 9, dans laquelle la méthode n'est pas pratiquée sur le corps humain ou animal.

12. Méthode pour inhiber sélectivement l'activité d'une première enzyme méthyltransférase en présence d'une deuxième enzyme méthyltransférase comprenant la mise en contact desdites enzymes méthyltransférases avec un composé selon l'une quelconque des revendications 1 à 9, dans laquelle la méthode n'est pas pratiquée sur le corps humain ou animal.

13. Composé selon l'une quelconque des revendications 1 à 9 pour utilisation dans le traitement du cancer.

14. Composé pour son utilisation selon la revendication 13 dans laquelle ledit cancer est le cancer du sein ou le cancer de la prostate.

15. Composé pour son utilisation selon la revendication 13 dans laquelle ledit cancer est une affection hématologique maligne, avantageusement la leucémie.
